# EUROPEAN PATENT SPECIFICATION

(11) **EP 3 840 705 B1**
(45) Date of publication and mention of the grant of the patent: **01.10.2025**
(21) Application number: 19762634.4
(22) Date of filing: 21.08.2019
(51) Int. Cl.: A61F 13/00, A61F 13/02, A61F 13/08, A61K 9/70, A61L 15/18

(54) **COMPRESSION BANDAGING**
KOMPRESSIONSBANDAGIERUNG
BANDAGE COMPRESSIF

(30) Priority: 22.08.2018 US 201862721222 P
(43) Date of publication of application: 30.06.2021
(73) Proprietor: Andover Healthcare, Inc., Salisbury, MA 01952 (US)
(72) Inventor: MURPHY, Thomas S., Salisbury, Massachusetts 01952 (US); BOYLE, James, Salisbury, Massachusetts 01952 (US)
(74) Representative: Hoffmann Eitle
(86) International application number: PCT/US2019/047506
(87) International publication number: WO 2020/041472

(56) References cited:
- EP-A1- 1 884 253
- EP-A2- 0 370 789
- US-A1- 2013 085 435
- US-A1- 2014 052 043

## Description

### 1. Field

The present invention relates generally to a compression bandage that includes a zinc oxide composition and/or one or more other therapeutic agents. In use, the bandage can be compression wrapped directly contacting a subject's skin for application of the zinc oxide composition and/or other therapeutic agents.

### 2. Background

Compression bandages are frequently used in medical and sports applications requiring a strong and reliable, yet comfortable and easily applied, means of securing a limb or other body segment for prolonged periods of time. For example, strains and sprains can cause inflammation and the accompanying accumulation of fluid around a sprained joint. Wrapping the affected joint securely with an elastic bandage can prevent excess fluid from accumulating and causing additional tissue damage.

US-A-2013/085435 discloses a wound care device for the treatment of wounds on a limb of a subject, said device comprising an inner, skin contacting layer formed of an elongate strip of a foam material, said foam material impregnated with a zinc oxide containing composition comprising zinc oxide in a pharmaceutically acceptable carrier.

US-A-2014/052043 relates to an elastic bandage for creating a compression dressing comprising foam and textile material having a textile inner layer that can be placed against the skin and at least one outer layer, characterized in that the inner layer has a woven, mesh and/or knit fabric.

EP-A-0 370 789 describes an occlusive composite compression and support dressing (10) comprising a woven, elasticated, extensible bandage (12) and a thin layer of a fluid-interactive hydrocolloid adhesive composition (14) laminated to one side of said bandage, wherein said fluid-interactive hydrocolloid adhesive composition comprises a substantially homogeneous mixture of 5-30 wt.% polyisobutylene optionally blended with butyl rubber, 3-30 wt.% styrene radial or block type copolymers, 8-40 wt.% mineral oil, 15-65 wt.% water soluble hydrocolloid gums, ≤ 15 wt.% water swellable cohesive strengthening agents, 7.5-15 wt.% tackifier, and ≤ 5 wt.% of optional ingredients selected from antioxidants, deodorants, medicaments, skin protective agents and reinforcing agents

EP-A-1 884 253 concerns an elastic antimicrobial material comprising synthetic elastic fibers woven with absorbent fibers and at least one biocidal agent, wherein the at least one biocidal agent is applied to the fibers after they are formed, and wherein a concentration of the at least one biocidal agent on the fibers is 0.01-0.1 mg/cm².

Certain tapes and bandages are available for such applications.

It would be desirable to have improved compression bandages.

### SUMMARY

We now provide new compression-type elastic bandages that include one or more agents for transdermal contact or administration to a subject. The present bandages can be suitably used as the sole bandage or material to treat a subject.

More particularly, the present invention provides a compression bandage comprising a stretchable, wrappable bandage material for contact on a subject's skin, which compression bandage comprises
(a) a polypropylene woven or nonwoven layer,
(b) an elastic layer comprised of transversely spaced, longitudinally extending coalesced multifilament elastic polyurethane strands,
(c) a warp-knitted, weft inserted polyester textile layer oriented with the knit yarns extending longitudinally and generally parallel to the orientation of the elastic strands,
all saturated and bound with a cohesive formula impregnated with a zinc oxide composition and/or one or more therapeutic agents.

Also, the present invention provides a kit comprising (1) the present compression bandage and (2) written instructions for use and/or identifying information of the compression bandage.

Preferred embodiments of the invention are as defined in the appended dependent claims and/or in the following detailed description.

In one aspect, the present compression bandage comprises one or more therapeutic agents other than a calamine composition, such as one or more antibiotics, antibacterial agents, antimicrobial agents, anti-odor agents, anti-inflammatory agent, anti-fungal agents, anti-viral agents, anti-cancer agents (including for treatment of a skin cancer and skin pre-cancers), eczema or psoriasis treatment agents, pain relieving agents, or combinations thereof. Preferably, such agents can be effectively administered transdermally to a subject.

Preferably the integration with the bandage material is such that the zinc oxide composition and/or therapeutic agents can readily contact and be administered to a subject skin upon application (for compression wrapping) of the bandage.

The compression bandage may be formed from a variety of materials and suitably may be a stretchable polymer composition as will be defined in more detail below. Suitable bandage materials include those that contain latex as well as those that are substantially or completely latex-free.

The compression bandage is suitably stretchable to facilitate applying a compressive force on a subject during use, i.e. a subject's limb, torso, head can be wrapped with the bandage and a compressive force thereby applied. In certain embodiments, the bandage material may have a maximum stretched length that is at least 30, 40, 50, 60, 70, 80, 90 or 100 % or more greater than the material's unstretched length. In some preferred materials, the bandage material may have a maximum stretched length that is at least 40 to 80, 90 or 100 % or more greater than the material's unstretched length.

The present compression bandages may be wound into a roll, e.g., for convenient storage and transport prior to use.

Methods for treating a subject suitably include circumferentially wrapping a subject with a compression bandage as disclosed herein, where the bandage directly contacts subject's skin. Thus, in these methods, a cushioned or absorbent material, or other material is not utilized and is thus not interposed between the present compression bandage and the subject's skin. Rather, the compression bandage is applied directly on and directly contacts the subject's skin. In certain applications, it may be desirable to apply another distinct material (e.g. a sleeve or stocking layer) over the wrapped compression bandage.

A wide variety of subjects suitably may be identified to utilize the present compression bandage. Thus, for instance, a person suffering from a sprain, strain or bruise, e.g. a joint or muscle sprain, strain or bruise, may experience relief by the compression force provided by a wrapped bandage as disclosed herein. Additionally, a zinc oxide composition, particularly calamine, present with the bandage can provide a favorable sensation to the subject's skin. Other therapeutic agents that may be present with the bandage also can provide a favorable effect for the subject. In particular, such one or more other therapeutic agents may be effectively administered transdermally to the subject.

In certain aspects, the subject will not be suffering from, at least in the skin area on which the compression bandage is applied, from any type of wound, such as skin ulcer, cancer (such as skin cancer or skin pre-cancer) psoriasis, eczema, or infection.

In other aspects, the subject may have one or more of e.g. such a wound, skin ulcer, psoriasis, eczema, cancer (including skin cancer or skin pre-cancer), or infection, and the present compression bandage is applied directly on the injury or distressed skin area.

Kits are also provided that contain a compression bandage as disclosed herein. Suitably, a kit also may contain written materials for use and/or identification of the compression bandage. The compression bandage is suitably packaged in roll form, i.e. where a strip of the bandage such as between 2.5 and 15 or more cm (between 1 and 6 or more inches) wide, such as 2.5, 5.1, 7.6, 10.2, 12.7 or 15.2 cm (1, 2, 3, 4, 5 or 6 inches) wide is wound and stored as a roll such as in lengths of at least one yard, more typically 1.83, 2.74, 3.66, 4.57, 5.49, 6.40, 7.32, 8.23, 9.14 m (2, 3, 4, 5, 6, 7, 8, 9, 10 yards) or more.

As referred to herein, the term zinc oxide containing composition is intended to include any composition containing zinc oxide as an active ingredient and which is suitable for impregnating the bandage material. The zinc oxide containing compositions herein may include calamine, ichthammol, or both. In certain aspects, the zinc oxide containing compositions may optionally include an antimicrobial agent and/or may be used in the presence of or the absence of an antimicrobial agent. If an antimicrobial agent is present, either as a part of the zinc oxide containing composition or as a separately applied antimicrobial agent or formula, such antimicrobial agent may be any antimicrobial agent suitable for topical administration, including organic or inorganic antimicrobial agents.

Terms such as stretchable bandage, elastic bandage, elastic layer, compression bandage, compression layer, and similar terms are intended to encompass both short stretch bandages and long stretch bandages unless specifically stated otherwise.

Other aspects of the invention are discussed infra.

### DETAILED DESCRIPTION

As discussed, new compression-type elastic bandages are provided that are adapted for direct skin contact. In preferred aspects, in use on a subject's leg (e.g. ankle, foot, lower leg, knee, upper leg), torso, arm (e.g. hand, forearm, elbow, upper arm), neck, or head, the bandage is circumferentially wrapped around the selected region of the subject, and the bandage directly (no interposing material) contacts the subject's skin for the complete circumference of that region of the subject, e.g. the complete circumference of the subject's leg (e.g. any of ankle, foot, lower leg, knee, upper leg), torso, arm (e.g. any of hand, forearm, elbow, upper arm), neck, or head,

The bandage material comprises one or more agents for contact to a subject's skin.

A zinc oxide composition, particularly calamine, is a preferred agent incorporated with a bandage material for contact to a subject's skin. Calamine may include zinc oxide and ferric oxide.

Additional preferred agents incorporated with a bandage material for contact to a subject's skin include one or more of antibiotic, an antibacterial agent, an antimicrobial agent, an anti-odor agent, an anti-inflammatory agent, an anti-fungal agent, an anti-viral agent, an anti-cancer agent (including for treatment of a skin cancer and skin pre-cancers), an eczema or psoriasis treatment agent, a pain relieving agent, or combination thereof.

More specifically, preferred additional agents incorporated with a bandage material for contact to a subject's skin include one or more of aloe; menthol; mupirocin; witch hazel, tree tea oil; clindamycin; anthralin; clotrimazole; ketoconazole; terbinafine; benzoyl peroxide; coal tar; corticosteroids; crisaborole; retinoids; salicylic acid; acyclovir; 5-fluorouracil; 5-fluorouracil, diclofenac; hyaluronic acid (alone or together with diclofenac); imiquimod; ingenol mebutate; adalimumab; and eucrisa.

Suitable anti-odor agents incorporated with a bandage material for contact to a subject's skin include, among others, activated carbon, cyclodextrins/modified cyclodextrins, activated alumina, metal powders, alumina silicates, metal oxides, zeolites, ceramics, diatomaceous earth, macroporous polymers, aerogels, cellulose and cellulosic derivatives, starches and starch derivatives, clay, talc, sodium bicarbonate, silicon dioxide, and combinations thereof.

Suitable antimicrobial agents incorporated with a bandage material for contact to a subject's skin include, among others, any appropriate antimicrobial composition useful for the intended purpose of preventing or inhibiting the growth or reproduction of microbes, such as bacteria, fungi, viruses , or protozoa, for example, selected from Beta Hemolytic *Streptococci* (*Streptococcus pyogenes*), *Enterococci* (*Enterococcus faecalis), Staphylococci (Staphylococcus aureus*/*MRSA), Pseudomonas aeruginosa, Enterobacter* species, *Escherichia coli, Klebsiella* species, *Proteus* species, *Bacteroides* species, *Clostridium* species, *Candida* specie *s, Aspergillus* species. In preferred embodiments, the antimicrobial agent may be e.g. inorganic metal based/organic antimicrobial agents, although it will be recognized that the antimicrobial agent may other antimicrobial agents as known in the art, including antibiotics, antiseptics, antiviral agents, antifungal agents, and disinfectants. In especially preferred embodiments, the antimicrobial agent is silver nanoparticles or silver nitrate.

Suitable antibiotic and antibacterial agents incorporated with a bandage material for contact to a subject's skin include, among others Methicillin, Neomycin sulfate, Bacitracin, Neomycin sulfate, and polymyxin B sulfate.

In certain preferred embodiments, the bandage material will comprise one or more agents for treatment of skin cancers and skin pre-cancers such as hypertrophic solar keratoses (including dysplastic hypertrophic solar keratosis and bowenoid hypertrophic solar keratosis), actinic keratosis and squamous cell carcinoma. In particular, for treatment of such skin cancers and pre-cancers, the bandage material may include one or more of 5-fluorouracil, diclofenac and/or hyaluronic acid, imiquimod and ingenol mebutate.

**In** certain methods of the invention, a subject suffering from or susceptible to a particular disease or disorder will be identified and selected to use a particular compression bandage.

For instance, a subject that has suffered from a strain or sprain (such as a strain or sprain of e.g. an ankle, elbow, knee, lower leg, upper leg, forearm, and upper arm) may be identified and selected, and that identified and selected subject's sprained/strained area may be wrapped with a compression bandage comprising calamine as disclosed herein.

A subject that is suffering from a skin cancer or pre-cancer such as hypertrophic solar keratoses (including dysplastic hypertrophic solar keratosis and bowenoid hypertrophic solar keratosis), actinic keratosis and/or squamous cell carcinoma may be identified and selected, and a compression bandage comprising one or more anticancer agents may be wrapped around the skin cancer regions of the selected subject. The one or more anticancer agents present in the compression bandage may be for example 5-fluorouracil, diclofenac and/or hyaluronic acid, imiquimod and ingenol mebutate.

Preferred bandage materials may be in strip or tape form and relatively easy to tear to any length by hand across the width, but at the same time should have sufficient longitudinal strength. CoFlex tapes and bandages (Andover Healthcare, Salisbury, MA; see. US 3,575,782) may be suitably employ and have a laminated structure including two outer nonwoven layers with longitudinally-extending elastic yarns sandwiched between. POWERFLEX bandages (Andover Healthcare; see US 5,762,623) also may be suitably employed as the bandage material and include a layer that is elastic in the longitudinally-extending direction laminated to one side of a warp-knitted, sometimes referred to as warp-knitted (weft insertion), fabric oriented with the knit yarns extending longitudinally.

In some embodiments, the bandage material is a "short stretch" elastic bandage and preferably a short stretch cohesive elastic bandage, which cohesively bonds to itself when wrapped in overlapping fashion, thus eliminating the need for bandage clips or other bandage fasteners. As used herein, the term "short stretch" refers to a bandage which is able to stretch, e.g., 25-80% beyond its original, unstretched length. Unlike "long stretch" articles, which are able to stretch from 100% up to several times beyond their original length in the longitudinal direction and thereby provide constant pressure at rest and work, i.e., a low static stiffness index (SSI), short stretch bandages are able to provide more effective compression through a low resting pressure and a high standing or working pressure, i.e., a high SSI, e.g., greater than 1.33 kPa (10 mm Hg). The use of a low stretch compression bandage as the outer layer is especially advantageous for patients with venous insufficiency, since the low resting pressure provides comfort when the patient is recumbent while also preventing expansion of muscle diameter while the patient is ambulatory, thereby increasing venous and lymphatic return when the muscles of the leg contract.

In certain embodiments, the bandage material may be a latex-free cohesive elastic bandage, as disclosed in US 6,156,424. In further embodiments, the bandage material may be a layer that eliminates the need for bandage scissors by facilitating hand tearing, as disclosed in US. 5,762,623.

One suitable bandage material is available from Andover Healthcare, Inc. (Salisbury, Mass.) under the trade designation "CoFlex NL." CoFlex NL is intended for controlled compression that will not constrict over time, i.e., a short-stretch bandage.

Optionally, an additional outermost layer such as a stocking, tube, or sleeve, preferably a nylon stocking, may be provided and worn over the wrapped bandage material, to provide a desired outward or cosmetic appearance of the bandage system.

Preferably, a zinc oxide composition and/or other one or more therapeutic agents are distributed throughout the bandage material. Such distribution can provide more extended application of calamine or other zinc oxide composition or other therapeutic agent(s) to the subject's skin around which the bandage material is wrapped.

Alternatively, a zinc oxide composition and/or other one or more therapeutic agents may be present only in selected regions of a bandage material, for example only within spaced strip areas that extend across the width and/or along the length of the bandage material. In one specific design, one or more polymer strips that contain a zinc oxide composition and/or other one or more therapeutic agents may be affixed (e.g. laminated) along the length or width of the bandage material.

A bandage material may be readily prepared. For instance, a formed bandage material may be impregnated with a zinc oxide containing composition or a composition containing one or more other therapeutic agents by dipping the bandage material in a bath containing the zinc oxide containing composition. The dipped bandage material then be formed into a roll at any desired length, width, and winding tension.

A mixture of polymer and a zinc oxide containing composition or a composition containing one or more other therapeutic agents also may be prepared. See, for instance, Example 1 which follows. That mixture then may be used to form a stretchable bandage material that includes a zinc oxide containing composition or a composition containing one or more other therapeutic agents.

The present compression bandage includes multi-layers, i.e. one or more woven or nonwoven textile layer(s), an inner elastic layer and a knit textile layer all saturated and bound with a cohesive formula impregnated with a zinc oxide composition such as calamine and/or one or more other therapeutic agents as disclosed herein. In a preferred embodiment, the woven or nonwoven layer is an 8-10 g/m², spun bound material such as polypropylene, polyethyleneteraphthalate or other material. Polypropylene is often preferred. Other polymeric woven or nonwovens may also be suitable. Although the preferred weight is 8-10 g/m², other weights may be suitable. A preferred elastic layer may be comprised of transversely spaced, longitudinally extending coalesced multifilament elastic polyurethane strands such as Lykra or Spandex. In one preferred aspect, suitably the denier of the yarns is 210 and the spacing is 2-5 per cm (5-15 per inch). Other deniers and spacing also may be suitable. The knit textile layer suitably is a warp-knitted (weft insertion) polyester textile oriented with the knit yarns extending longitudinally and generally parallel to the orientation of the elastic strands. Other polymeric or natural fiber warp knits may be suitable as well. The warp-knitted (weft insertion) textile suitably has a weight of less than 50 g/m² (e.g., 1.5 oz/yd²) and most preferably less than 25-30 g/m² (0.7-0.9 oz/yd²). Preferred fibers include those that are 5 x 5 fibers per cm (12 x 12 fibers per inch), although other constructions may be suitable. The layers described above are then laminated with a cohesive formula that includes a zinc oxide composition and/or one or more other therapeutic agents as disclosed herein and metered to the desired coat weight. The cohesive formula's base polymer suitably can be, for example, natural rubber latex, polychloroprene or polyurethane. In the formula, there suitably may be one or more tackifying resins, surfactants, fillers, cross linkers, pigments, thixotropes, and preservatives. The zinc oxide composition such as calamine and/or one or more other therapeutic agents as disclosed herein are suitably added to the cohesive formula. Such incorporating of the zinc oxide composition and/or one or more other therapeutic agents can result in the zinc oxide composition and/or other therapeutic agent(s) being substantially homogenous throughout the bandage material. As a consequence, in preferred aspects, the bandage material can be wound on the patient in either direction onto the skin. Preferred resulting composite bandages can have a good appearance and hand-tear cleanly transversely across the bandage.

The following examples are illustrative of the invention

### Example 1:

A suitable compression bandage with added agent (calamine) can be prepared as follows as set forth in the following Examples 1 and 2.

Begin by adding polychloroprene dispersion (25.878 kg (57.050 lb)) to a clean vessel. When the polychloroprene reaches the bottom of the blade, turn on the mixer. Add Serdas 7005 defoamer (0.005 kg (0.01 11b)). Finish adding the balance of the polychloroprene. Add Snowtack 880G-rosin ester (6.310 kg (13.910 lbs)). Add Aquatac 6085 rosin ester (9.852 kg (21.720 lbs)) through an 800-micron filter. Add Bostex 24 antioxidant (0.493 kg (1.086 lbs)). Add Rovene 4301 carboxylated styrene butadiene (0.197 kg (0.434 lbs)). Sift in Calamine (2.552 kg (5.625 lbs)) using hopper or hand sifting. Add pigments per color specification. Red (0.020 kg (0.043 lb) and neon yellow (0.059 kg (0.130 lb)) for Calamine color. Continue mixing until use to form the stretchable bandage material.

### Example 2:

A preferred compressions bandage system is provided by an 8-10 g/m², spun bound polypropylene nonwoven with an elastic layer of transversely spaced, longitudinally extending coalesced multifilament Lykra or Spandex. The denier of the yarns is 210 and the spacing is 2-5 per cm (5-15 per inch). The knit textile layer is a warp-knitted (weft insertion) polyester textile oriented with the knit yarns extending longitudinally and generally parallel to the orientation of the elastic strands. The warp-knitted (weft insertion) textile has a weight of less than 50 g/m² (1.5 oz/yd²) and most preferably less than 25-30 g/m² (0.7-0.9 oz/yd²). The preferred fibers are 5 x 5 fibers per cm (12 x 12 fibers per inch). The layers described above are then laminated with a cohesive formula described in Example 1 above and metered to the desired coat weight.

## Claims

1. A compression bandage comprising a stretchable, wrappable bandage material for contact on a subject's skin, which compression bandage comprises
(a) a polypropylene woven or nonwoven layer,
(b) an elastic layer comprised of transversely spaced, longitudinally extending coalesced multifilament elastic polyurethane strands,
(c) a warp-knitted, weft inserted polyester textile layer oriented with the knit yarns extending longitudinally and generally parallel to the orientation of the elastic strands,
all saturated and bound with a cohesive formula impregnated with a zinc oxide composition and/or one or more therapeutic agents.

2. The compression bandage of claim 1, wherein the zinc oxide composition is calamine.

3. The compression bandage of claim 1 or 2 wherein the bandage material comprises one or more of an antibiotic, an antibacterial agent, an antimicrobial agent, an anti-odor agent, an anti-inflammatory agent, an anti-fungal agent, an anti-viral agent, an anti-cancer agent, an eczema or psoriasis treatment agent, a pain relieving agent, or combination thereof.

4. The compression bandage of any of claims 1-2 wherein the bandage material comprises
one or more of aloe, menthol, mupirocin, witch hazel, tree tea oil, clindamycin, anthralin, clotrimazole, ketoconazole, terbinafine, benzoyl peroxide, coal tar, corticosteroids, crisaborole, retinoids, salicylic acid, acyclovir, 5-fluorouracil, 5-fluorouracil, diclofenac, hyaluronic acid, imiquimod, ingenol mebutate, adalimumab and eucrisa,
and preferably one or more of 5-fluorouracil, diclofenac and/or hyaluronic acid, imiquimod and ingenol mebutate.

5. The compression bandage of any of claims 1-4 wherein the zinc oxide composition and/or therapeutic agents
- are distributed substantially uniformly throughout the bandage material, or
- reside in selected regions of the bandage material, and preferably are present in first regions of the bandage material and are not present in second regions of the bandage materials which are distinct from the first regions.

6. The compression bandage of any of claims 1-5 which has an unstretched length and a maximum stretched length, wherein the maximum stretched length is 40-80% greater than the unstretched length.

7. The compression bandage of any of claims 1-6 which is stretchable to provide a compressive force when wrapped about the limb of a subject.

8. The compression bandage of any of claims 1-7 wherein the bandage material comprises latex or is latex-free.

9. The compression bandage of any of claims 1-8 which is in a roll form, and preferably is 2.54-15.24 cm (1-6 inches) wide and rolled along the bandage material length.

10. A kit comprising (1) the compression bandage of any of claims 1-9 and (2) written instructions for use and/or identifying information of the compression bandage.

11. The kit of claim 10 which does not contain a separate, additional bandage material.

12. The kit of claim 10 or 11 which comprises a box unit comprising a roll of the compression bandage (1) and the written instructions (2).

13. The kit of any of claims 10-12 which comprises within sealed packaging a roll of the compression bandage (1) and the written instructions (2).

14. The kit of any of claims 10-13 which consists of a roll of the compression bandage (1) and the written instructions (2).

## Patentansprüche

1. Kompressionsverband, umfassend ein streckbares, wickelbares Verbandsmaterial zum Kontakt auf der Haut eines Patienten, wobei der Kompressionsverband umfasst
(a) eine gewebte oder nicht gewebte Polypropylenschicht,
(b) eine elastische Schicht, umfassend quer beabstandete, sich in Längsrichtung erstreckende, miteinander verbundene multifilamentäre elastische Polyurethanstränge,
(c) eine kettengewirkte, schussverstärkte Polyester-Textilschicht, deren Maschen sich in Längsrichtung und im Wesentlichen parallel zur Ausrichtung der elastischen Stränge erstrecken,
alle gesättigt und gebunden mit einer Kohäsivzusammensetzung, imprägniert mit einer Zinkoxidzusammensetzung und/oder einem oder mehreren therapeutischen Mitteln.

2. Kompressionsverband gemäß Anspruch 1, worin die Zinkoxidzusammensetzung Kalamin ist.

3. Kompressionsverband gemäß Anspruch 1 oder 2, worin das Verbandsmaterial eines oder mehrere von einem Antibiotikum, einem antibakteriellen Mittel, einem antimikrobiellen Mittel, einem Anti-Geruchsmittel, einem Entzündungshemmer, einem Fungizid, einem antiviralen Mittel, einem Krebsmittel, einem Ekzem- oder Psoriasis-Behandlungsmittel, einem Schmerzmittel oder einer Kombination hiervon umfasst.

4. Kompressionsverband gemäß irgendeinem der Ansprüche 1 bis 2, worin das Verbandsmaterial umfasst
eines oder mehr von Aloe, Menthol, Mupirocin, Hamamelis, Teebaumöl, Clindamycin, Anthralin, Clotrimazol, Ketocenazol, Terbinafin, Benzoylperoxid, Kohlenteer, Croticosteroide, Crisaborol, Retinoide, Salicylsäure, Acyclovir, 5-Fluoruracil, 5-Fluoruracil, Diclofenac, Hyaluronsäure, Imiquimod, Ingenolmebutat, Adalimumab und Eucrisa,
und bevorzugt eines oder mehr von 5-Fluoruracil, Diclofenac und/oder Hyaluronsäure, Imiquimod und Ingenolmebutat.

5. Kompressionsverband gemäß irgendeinem der Ansprüche 1-4, worin die Zinkoxidzusammensetzung und/oder therapeutische Mittel
- im Wesentlichen gleichmäßig in dem Verbandsmaterial verteilt sind oder
- in ausgewählten Regionen des Verbandsmaterials lokalisiert sind, und bevorzugt in ersten Bereichen des Verbandsmaterials vorliegen und in zweiten Bereichen des Verbandsmaterials nicht vorliegen, welche sich von den ersten Bereichen unterscheiden.

6. Kompressionsverband gemäß irgendeinem der Ansprüche 1-5, welches eine ungestreckte Länge und eine maximal gestreckte Länge aufweist, worin die maximal gestreckte Länge 40-80 % größer ist als die ungestreckte Länge.

7. Kompressionsverband gemäß irgendeinem der Ansprüche 1-6, welches streckbar ist, um eine Kompressionskraft bereitzustellen, wenn es um das Gliedmaß eines Patienten gewickelt wird.

8. Kompressionsverband gemäß irgendeinem der Ansprüche 1-7, worin das Verbandsmaterial Latex umfasst oder latexfrei ist.

9. Kompressionsverband gemäß irgendeinem der Ansprüche 1-8, welches in Rollenform vorliegt, und welcher bevorzugt 2,54-15,24 cm (1-6 Inches) breit ist und entlang der Länge des Verbandsmaterials aufgerollt ist.

10. Kit, umfassend (1) den Kompressionsverband gemäß irgendeinem der Ansprüche 1-9 und (2) schriftliche Instruktionen für die Verwendung und/oder zum Identifizieren von Information bezüglich des Kompressionsverbands.

11. Kit gemäß Anspruch 10, welches kein separates, zusätzliches Verbandsmaterial umfasst.

12. Kit gemäß Anspruch 10 oder 11, der eine Box-Einheit umfasst, umfassend eine Rolle des Kompressionsverbands (1) und die schriftlichen Instruktionen (2).

13. Kit gemäß irgendeinem der Ansprüche 10-12, welcher innerhalb einer verschlossenen Verpackung eine Rolle des Kompressionsverbands (1) und die schriftlichen Instruktionen (2) umfasst.

14. Kit gemäß irgendeinem der Ansprüche 10-13, der aus einer Rolle des Kompressionsverbands (1) und den schriftlichen Instruktionen (2) besteht.

## Revendications

1. Bandage compressif comprenant un matériau de bandage étirable et enroulable pour le contact sur la peau d'un sujet, le bandage compressif comprenant
(a) une couche tissée ou non tissée de polypropylène,
(b) une couche élastique composée de brins de polyuréthane élastiques multifilaments coalescents espacés transversalement et s'étendant longitudinalement,
(c) une couche textile de polyester indémaillable à trame insérée orientée avec les fils tricotés s'étendant longitudinalement et généralement parallèle à l'orientation des brins élastiques,
toutes saturées et liées avec une formule cohésive imprégnée d'une composition d'oxyde de zinc et/ou d'un ou plusieurs agents thérapeutiques.

2. Bandage compressif selon la revendication 1, dans lequel la composition d'oxyde de zinc est de la calamine.

3. Bandage compressif selon la revendication 1 ou la revendication 2 dans lequel le matériau de bandage comprend un ou plusieurs d'un antibiotique, d'un agent antibactérien, d'un agent antimicrobien, d'un agent anti-odeur, d'un agent anti-inflammatoire, d'un agent antifongique, d'un agent antiviral, d'un agent anti-cancer, d'un agent de traitement de l'eczéma ou du psoriasis, d'un agent soulageant la douleur, ou d'une combinaison de ceux-ci.

4. Bandage compressif selon l'une quelconque des revendications 1 à 2 dans lequel le matériau de bandage comprend un ou plusieurs d'aloès, de menthol, de mupirocine, d'hamamélis, d'huile d'arbre à thé, de clindamycine, d'anthraline, de clotrimazole, de kétoconazole, de terbinafine, de peroxyde de benzoyle, de goudron de houille, de corticoïdes, de crisaborole, de rétinoïdes, d'acide salicylique, d'acyclovir, de 5-fluorouracile, de diclofénac, d'acide hyaluronique, d'imiquimod, de mébutate d'ingénol, d'adalimumab et d'eucreas,
et de préférence un ou plusieurs de 5-fluorouracile, de diclofénac et/ou d'acide hyaluronique, d'imiquimod et de mébutate d'ingénol.

5. Bandage compressif selon l'une quelconque des revendications 1 à 4 dans lequel la composition d'oxyde de zinc et/ou d'agents thérapeutiques
- sont distribués substantiellement uniformément au sein du matériau de bandage, ou
- résident dans des régions sélectionnées du matériau de bandage, et de préférence sont présents dans des premières régions du matériau de bandage et ne sont pas présents dans des secondes régions du matériau de bandage qui sont distinctes des premières régions.

6. Bandage compressif selon l'une quelconque des revendications 1 à 5 qui présente une longueur non étirée et une longueur étirée maximale, dans lequel la longueur étirée maximale est 40 à 80 % supérieure à la longueur non étirée.

7. Bandage compressif selon l'une quelconque des revendications 1 à 6 qui est étirable pour fournir une force de compression lorsqu'il est enroulé autour du membre d'un sujet.

8. Bandage compressif selon l'une quelconque des revendications 1 à 7 dans lequel le matériau de bandage comprend du latex ou est exempt de latex.

9. Bandage compressif selon l'une quelconque des revendications 1 à 8 qui est sous une forme de rouleau, et de préférence d'une largeur de 2,54 à 15,24 cm et enroulé le long de la longueur du matériau de bandage.

10. Kit comprenant (1) le bandage compressif selon l'une quelconque des revendications 1 à 9 et (2) des instructions d'utilisation écrites et/ou des informations d'identification du bandage compressif.

11. Kit selon la revendication 10 qui ne contient pas un matériau de bandage séparé supplémentaire.

12. Kit selon la revendication 10 ou la revendication 11 qui comprend une unité de boîte comprenant un rouleau du bandage compressif (1) et les instructions écrites (2).

13. Kit selon l'une quelconque des revendications 10 à 12 qui comprend à l'intérieur d'un emballage scellé un rouleau du bandage compressif (1) et les instructions écrites (2).

14. Kit selon l'une quelconque des revendications 10 à 13 qui consiste en un rouleau du bandage compressif (1) et des instructions écrites (2).
